# EUROPÄISCHE PATENTSCHRIFT

(11) **EP 4 066 921 B1**
(45) Veröffentlichungstag und Bekanntmachung des Hinweises auf die Patenterteilung: **27.12.2023**
(21) Anmeldenummer: 21020173.7
(22) Anmeldetag: 31.03.2021
(51) Int. Cl.: B01D 53/14, B01D 53/22, C07C 29/151, C01C 1/04, C01B 3/02, C01B 3/38, C01B 3/36

(54) **VERFAHREN UND ANLAGE ZUM HERSTELLEN VON METHANOL UND AMMONIAK**
METHOD AND INSTALLATION FOR PRODUCING METHANOL AND AMMONIA
PROCÉDÉ ET INSTALLATION DE PRODUCTION DE MÉTHANOL ET D'AMMONIAQUE

(43) Veröffentlichungstag der Anmeldung: 05.10.2022
(73) Patentinhaber: L'Air Liquide, Société Anonyme pour l'Etude et l'Exploitation des Procédés Georges Claude, 75007 Paris (FR)
(72) Erfinder: Walter, Stefan, D-60439 Frankfurt am Main (DE); Haase, Stephan, D-60439 Frankfurt am Main (DE); Cortale, Manon, F-94503 Champigny-sur-Marne (FR); Schmidt, Sophia, D-60439 Frankfurt am Main (DE)
(74) Vertreter: Dropsch, Holger

(56) Entgegenhaltungen:
- EP-A1- 2 196 448
- WO-A1-2005/095313

## Beschreibung

### Gebiet der Erfindung

Die Erfindung betrifft ein Verfahren zur parallelen Herstellung von Methanol und Ammoniak durch heterogen-katalysierte Umsetzung von Wasserstoff und Kohlenoxiden einerseits bzw. Wasserstoff und Stickstoff andererseits in entsprechenden, an sich bekannten Synthesereaktoren, wobei der Schwerpunkt der Erfindung auf der Herstellung, Konditionierung und optimierten stofflichen Nutzung des hierfür erforderlichen Synthesegases liegt. Die Erfindung betrifft ferner eine Anlage zur Durchführung eines solchen Herstellungsverfahrens.

### Stand der Technik

Verfahren zur industriellen Herstellung von Methanol und Ammoniak durch heterogenkatalytische Umsetzung von Synthesegas bzw. des hierin enthaltenen Wasserstoffs in geeigneten Synthesereaktoren sind der Fachwelt seit langem bekannt. Als Synthesegase bezeichnet man dabei Wasserstoff und Kohlenoxide enthaltende Gasgemische, die in verschiedenen Synthesereaktionen Verwendung finden.

Beide Stoffe stellen wichtige, unverzichtbare Basischemikalien der chemischen Industrie für die Weiterverabreitung zu Endprodukten dar. In Ullmann's Encyclopedia of Industrial Chemistry, Sixth Edition, 1998 Electronic Release, Kapitel "Methanol", Unterkapitel 5 "Process Technology" und Kapitel "Ammonia", Unterkapitel 4 "Production" werden verschiedene Grundverfahren zur Herstellung der genannten Stoffe beschrieben.

Ein moderneres, zweistufiges Verfahren zur Herstellung von Methanol ist beispielsweise aus der europäischen Patentschrift EP 0 790 226 B1 bekannt. Das Methanol wird in einem Kreisprozess hergestellt, bei dem eine Mischung von frischem und teilweise reagiertem Synthesegas zunächst einem wassergekühlten Reaktor (WCR) und dann einem gasgekühlten Reaktor (GCR) zugeführt wird, in welchen das Synthesegas jeweils an einem kupferbasierten Festbett-Katalysator zu Methanol umgesetzt wird. Das in dem Prozess hergestellte Methanol wird von dem zurückzuführenden Synthesegas abgeschieden, welches dann als Kühlmittel im Gegenstrom durch den gasgekühlten Reaktor hindurchgeführt und auf eine Temperatur von 220 bis 280 °C vorgewärmt wird, bevor es in den ersten Synthesereaktor eingebracht wird. Ein Teil des zurückzuführenden Synthesegases wird als Spülstrom (sog. Purge) aus dem Verfahren entfernt, um zu verhindern, dass sich Inertkomponenten innerhalb des Synthesekreislaufes anreichern.

Als Inertkomponente im Sinne der Methanolsynthese und auch der Ammoniaksynthese wird dabei nicht umgesetztes Methan aus der Synthesegaserzeugung betrachtet, da sich dieses unter den Bedingungen der Methanol- bzw. Ammoniaksynthese ebenfalls nicht weiter umsetzt. Dasselbe gilt für Argon, das über Feedströme in die Synthesegaserzeugung gelangt.

Ein aktuelles Verfahren zur Ammoniaksynthese wird beispielsweise in der Patentveröffentlichung WO 2002/038499 A1 beschrieben. Im Vergleich zu dem für die Methanolsynthese verwendeten Synthesegas ist es dabei wichtig, bei dem Synthesegas für die Ammoniaksynthese den Anteil an Kohlenoxiden vollständig zu eliminieren, so dass Wasserstoff als allein verbliebener Synthesegasbestandteil in die Ammoniaksynthese gelangt. Dies erfolgt zunächst durch die Konvertierung des im Synthesegas enthaltenen Kohlenmonoxids (CO-Konvertierung), einer sich an diese anschließende Kohlendioxidentfernung mittels eines Sorptionsverfahrens und schließlich mittels kryogener Gaszerlegung.

Es existieren unterschiedliche Verfahren zur Herstellung von Wasserstoff und Kohlenoxide umfassendem Synthesegas als Einsatzgas für die Methanolsynthese und die Ammoniaksynthese, beispielsweise die Dampfreformierung, die autotherme Reformierung (ATR), Kombinationen beider (sog. Combined Reforming) und die nichtkatalytische Partialoxidation (POX). Technische Einzelheiten dieser Verfahren sind der Fachwelt bekannt und werden beispielsweise in Ullmann's Encyclopedia of Industrial Chemistry, Sixth Edition, 1998 Electronic Release, Stichwort "Gas Production", im Detail erläutert.

Als besonders häufig realisierte Variante der Dampfreformierung ist die Dampfreformierung von Erdgas als Einsatzgas zu nennen. Wegen des hohen Methangehalts im Erdgas wird dann auch von Steam Methane Reforming (SMR) gesprochen.

Eine weitere Variante der Dampfreformierung besteht darin, die mit Katalysator gefüllten Reaktorrohre, auch als Spaltrohre bezeichnet, nicht mittels Brennerflammen durch Wärmestrahlung zu beheizen, sondern heiße Rauchgase oder heißes Synthesegas aus einer nachgeschalteten Reformierungsstufe, beispielsweise aus einem ATR, für die Beheizung der Reaktorrohre zu verwenden, um auf diese die für die endotherm verlaufende Dampfspaltung benötigte Energie zu übertragen. Die Wärmeübertragung verläuft dabei weitgehend konvektiv und der entsprechende Reformertyp wird als gasbeheizter Reformer (Gas Heated Reformer, GHR) bezeichnet.

Als Ausgangsstoffe der oben genannten Verfahren zur Synthesegaserzeugung dienen dabei Kohlenwasserstoffe wie Erdgas mit seiner Hauptkomponente Methan oder Naphtha. Die genannten Verfahren liefern unterschiedliche Verhältnisse der Produktkomponenten Kohlenmonoxid (CO) und Wasserstoff (H₂), wie anhand der folgenden Reaktionsgleichungen ersichtlich ist:

| | | |
|---|---|---|
| 2 CH₄ + O₂ | = 2 CO + 4 H₂ | (Partialoxidation) |
| 2 CH₄ + ½ O₂ + H₂O | = 2 CO + 5 H₂ | (autotherme Reformierung) |
| 2 CH₄ + 2 H₂O | = 2 CO + 6 H₂ | (reine Dampfreformierung) |

Da bei der Partialoxidation oder der autothermen Reformierung mit einem Überschuss an Kohlenwasserstoff bzw. Unterschuss an Sauerstoff gearbeitet wird, um die Totaloxidation des Kohlenwasserstoffs zu Kohlendioxid zu unterdrücken, wird oft ein Synthesegas erhalten, dass in Bezug auf seine Verwendung als Einsatzgas für die Methanolsynthese ein Wasserstoffdefizit aufweist. Diese erfordert gemäß folgender Reaktionsgleichung

2 H₂ + CO = CHsOH

ein H₂/CO-Verhältnis von mindestens 2, unter praktischen Synthesebedingungen oft sogar etwas größer als 2, beispielsweise 2,1. Dieses Verhältnis wird üblicherweise als Stöchiometriezahl SN der Methanolsynthese formuliert, die berücksichtigt, dass auch Kohlendioxid zu Methanol reagiert:

SN = ([H₂] - [CO₂]) / ([CO] + [CO₂]) ≥ 2 (z. B. 2,1)

Mittels Partialoxidation oder autothermer Reformierung erhaltene Synthesegase weisen dagegen oft eine Stöchiometriezahl von ≤ 1,9, gelegentlich sogar ≤ 1,7 auf. Somit wird von keinem der Reformierungs- bzw. Partialoxidationsverfahren für sich betrachtet ein Synthesegasprodukt mit dem für die Methanolsynthese gewünschten, stöchiometrischen H₂/CO-Verhältnis von 2 bzw. mit nur leichtem Wasserstoff-Überschuss erhalten.

Ferner ist es im Hinblick auf die parallel durchzuführende Ammoniaksynthese erforderlich, in dem hierfür als Feed bestimmten Anteil des Synthesegases Kohlenoxide abzutrennen und den Anteil des Wasserstoffs zu maximieren. Dies erfolgt üblicherweise mittels der CO-Konvertierungsreaktion, auch als Wassergas-Shift-Reaktion (WGS) oder CO-Shift-Reaktion bezeichnet, gemäß der Umsatzgleichung

CO + H₂O = CO₂ + H₂

Unter Zugabe von Wasserdampf reagiert demnach das CO zu CO₂ und H₂. Je nach angewandter Reaktionstemperatur wird dabei von Hochtemperatur (HTS)-, Mitteltemperatur (MTS)- oder Tieftemperatur (LTS)-Shift gesprochen.

Die weitere Aufbereitung des erzeugten Rohsynthesegases umfasst zumeist auch ein Sorptionsverfahren zur Abtrennung weiterer unerwünschter Begleitstoffe, beispielsweise mittels physikalischer oder chemischer Absorption oder Gaswäsche. So können mit solchen Verfahren unerwünschte Bestandteile, insbesondere Kohlendioxid (CO₂), von den erwünschten Synthesegas-Hauptbestandteilen Wasserstoff und Kohlenmonoxid sicher bis in den Spurenbereich entfernt werden. Ein bekanntes und häufig angewendetes Verfahren ist das Rectisol-Verfahren, das eine Wäsche des Rohsynthesegases mit tiefkaltem Methanol als Absorbens umfasst und ebenfalls im oben genannten Schrifttum grundsätzlich beschrieben wird.

Zur Entfernung von Spuren höherer Kohlenwasserstoffe oder von Kohlenmonoxid kann die kryogene Gaszerlegung (sog. Coldbox) eingesetzt werden. Bei dieser wird hauptsächlich flüssiges Methan oder flüssiger Stickstoff verwendet, um höher siedende Gase wie Kohlenmonoxid zu absorbieren. Zur Aufbereitung des für die Ammoniaksynthese erforderlichen Wasserstoffs wird üblicherweise eine Flüssigstickstoffwäsche durchgeführt, bei der vorteilhafterweise eine Wasserstoff/Stickstoff-Gemisch mit dem für die Ammoniaksynthese idealen Verhältnis von 3 mol/mol gewonnen wird. Der hierbei erhaltene Abgasstrom kann als Heizgas verwendet werden oder alternativ mittels weiterer kryogener Gaszerlegung in einen methanreichen Gasstrom und in einen weiteren, Kohlenmonoxid und Wasserstoff umfassenden Gasstrom aufgetrennt werden, falls dies erwünscht oder erforderlich ist.

Ein Verfahren zur kombinierten Synthese von Ammoniak und Methanol wird beispielsweise in der Patentveröffentlichung WO 2005/095313 A1 beschrieben. Nachteilig ist es hierbei, dass der als Strom 6 zu der Methanolsynthese zurückgeführte, Wasserstoff enthaltende Gasstrom dem Reinwasserstoffprodukt der Reinigungseinheit D entnommen wird, so dass er für die Ammoniaksynthese nicht mehr zur Verfügung steht. Ferner wäre es wünschenwert, weitere in diesem Verfahren anfallende Abfallstöme nicht nur thermisch als Brennstoff, sondern auch stofflich zu nutzen.

### Beschreibung der Erfindung

Die Aufgabe der vorliegenden Erfindung besteht daher darin, ein Verfahren und eine Anlage anzugeben, das die beschriebenen Nachteile des Stands der Technik nicht aufweist und das es insbesondere ermöglicht, in einem Verfahren zur parallelen Erzeugung von Methanol und Ammoniak möglichst alle anfallenden Stoffströme bevorzugt stofflich zu nutzen. Ferner soll mit der Erfindung eine optimale Einstellung der Stöchiometriezahl für die Methanolsynthese ohne Import von nicht im Verfahren erzeugtem Wasserstoff ermöglicht werden.

Diese Aufgabe wird in einem ersten Aspekt der Erfindung durch ein Verfahren mit den Merkmalen des Anspruchs 1 und durch eine Anlage mit den Merkmalen des Anspruchs 9 gelöst. Weitere Ausgestaltungen nach weiteren Aspekten der Erfindung ergeben sich aus den Unteransprüchen der jeweiligen Kategorie.

Unter Synthesegaserzeugungsbedingungen, Methanolsynthesebedingungen, CO-Konvertierungsbedingungen, Ammoniaksynthesebedingungen sind die dem Fachmann an sich bekannten Verfahrensbedingungen, insbesondere von Temperatur, Druck, Verweilzeit, zu verstehen, wie sie oben beispielhaft genannt und detailliert im einschlägigen Schrifttum erörtert werden und bei denen mindestens ein Teilumsatz, bevorzugt allerdings technisch relevante Umsätze der Edukte zu den Produkten des jeweiligen Verfahrens erfolgt. Dasselbe gilt für die Auswahl eines geeigneten Katalysators und geeigneter Betriebsbedingungen desselben, da im Sinne der vorliegenden Erfindung alle genannten Verfahren heterogen-katalytisch betrieben werden, mit Ausnahme der Partialoxidation (POX). Entsprechende Synthesegaserzeugungsreaktoren, Methanolsynthesereaktoren, CO-Konvertierungsanlagen und Ammoniaksynthesereaktoren sind der Fachwelt an sich bekannt und beispielsweise in dem eingangs erwähnten Schrifttum beschrieben.

Unter einer Sorptionsvorrichtung wird im Rahmen der vorliegenden Offenbarung eine Vorrichtung verstanden, die es ermöglicht, dass ein Fluidgemisch, beispielsweise ein Gasgemisch, mittels eines physikalischen oder chemischen Sorptionsverfahrens unter Verwendung eines geeigneten Sorbens in seine Bestandteile aufgetrennt bzw. unerwünschte Komponenten aus diesem Gemisch abgetrennt werden können. Das Sorptionsverfahren kann dabei auf einer Adsorption, also einer Bindung des oder der abzutrennenden Stoffe an eine Oberfläche oder Grenzfläche des festen Adsorbens, oder auf einer Absorption, also einer Aufnahme des oder der abzutrennenden Stoffe in das Volumen des flüssigen oder festen Absorbens, beruhen. Der oder die abgetrennten und mittels Sorption gebundenen Stoffe werden als Ad- bzw. Absorbat bezeichnet. Die dabei wirkenden Bindungskräfte können physikalischer oder chemischer Art sein. Entsprechend wirken bei der physikalischen Sorption zumeist schwächere, unspezifischere Bindungskräfte, z. B. van-der-Waals-Kräfte, wohingegen bei der chemischen Sorption stärkere, spezifischere Bindungskräfte wirken und das Ad- bzw. Absorbat und/oder das Ad- bzw. Absorbens chemisch verändert wird.

Ein spezifisches, physikalisches Absorptionsverfahren stellt die Gaswäsche mit tiefkaltem Methanol dar, das als Absorbens oder Waschmittel Methanol verwendet, dessen Temperatur mittels kälteerzeugender Verfahren unter die Umgebungstemperatur, bevorzugt unter 0 °C, meist bevorzugt unter - 30 °C abgekühlt wurde. Dieses Verfahren ist dem Fachmann unter der Bezeichnung Rectisol-Verfahren bekannt.

Unter einem Aufteilen eines Stoffstroms ist im Zusammenhang mit der vorliegenden Erfindung ein Aufspalten des Stroms in mindestens zwei Teilströme zu verstehen, deren stoffliche Zusammensetzung und Phasenzustand derjenigen des Ausgangsstroms entspricht. Im Gegensatz hierzu ist mit dem Auftrennen eines Stoffstroms die Aufspalten des Stroms in mindestens zwei Teilströme unter Zuhilfenahme eines Phasengleichgewichts gemeint, wobei sich die Zusammensetzung der erhaltenen Stoffströme untereinander und von derjenigen des Ausgangsstroms unterscheidet.

Flüssigstickstoffwaschstufen sind an sich bekannt und werden beispielsweise in Häring, H. W., Industrial Gases Processing, WILEY-VCH Verlag, Weinheim (2008), S. 156 beschrieben. Flüssigstickstoffwaschstufen im Sinne der Erfindung sind insbesondere solche Vorrichtungen, in denen der erhaltene Abgasstrom mittels weiterer kryogener Gaszerlegung in einen methanreichen Gasstrom und in einen weiteren, Kohlenmonoxid und Wasserstoff umfassenden Gasstrom aufgetrennt wird, wovon optional auch in der internationalen Patentanmeldung WO 2002/038499 A1 ausgegangen wird.

Unter einem Hauptbestandteil eines Stoffstroms werden Komponenten verstanden, deren Anteil größer als 1 Vol.-%, bevorzugt größer als 10 Vol.-% beträgt und die somit als wichtigste und vorherrschende Komponenten des Stoffstroms zu betrachten sind und die physikalisch-chemischen Eigenschaften des Stoffstroms im Wesentlichen definieren. Dagegen werden als Spurenbestandteile eines Stoffstroms Komponenten verstanden, deren Anteil kleiner als 1 Vol.-% beträgt.

Unter der Angabe, dass ein Stoffstrom überwiegend aus einer Komponente oder Komponentengruppe besteht, ist zu verstehen, dass der Stoffmengenanteil (Molenbruch) oder Volumenanteil dieser Komponente oder Komponentengruppe mengenmäßig größer ist als alle anderen jeweils für sich betrachteten Anteile anderer Komponenten oder Komponentengruppen in dem Stoffstrom. Insbesondere bei binären Gemischen ist darunter ein Anteil von mehr als 50 % zu verstehen. Sofern im konkreten Fall nichts anderes angegeben ist, wird hierbei der Volumenanteil zugrunde gelegt. Im Einklang hiermit wird unter einem kohlendioxidreichen Strom ein Stoffstrom verstanden, bei dem Kohlendioxid-Anteil mengenmäßig größer ist als alle anderen jeweils für sich betrachteten Anteile anderer Komponenten in dem Stoffstrom und insbesondere mehr als 50 Vol.-%, bevorzugt mehr als 70 Vol.-%, meist bevorzugt mehr als 90 Vol.-% beträgt.

Unter einem Mittel wird eine Sache verstanden, die die Erreichung eines Zieles ermöglicht oder dabei behilflich ist. Insbesondere werden unter Mitteln zum Durchführen eines bestimmten Verfahrensschrittes alle diejenigen physischen Gegenstände verstanden, die der Fachmann in Betracht ziehen würde, um diesen Verfahrensschritt durchführen zu können. Beispielsweise wird der Fachmann als Mittel zum Einleiten oder Ausleiten eines Stoffstroms alle Transport- und Fördervorrichtungen, also z. B. Rohrleitungen, Pumpen, Verdichter, Ventile sowie die entsprechenden Durchtrittsöffnungen in Behälterwänden in Betracht ziehen, die ihm aufgrund seines Fachwissens zur Durchführung dieses Verfahrensschrittes notwendig oder sinnvoll erscheinen.

Unter Fluidverbindung zwischen zwei Bereichen oder Anlagenteilen wird dabei jegliche Art von Verbindung verstanden, die es ermöglicht, dass ein Fluid, beispielsweise ein Reaktionsprodukt oder eine Kohlenwasserstofffraktion, von dem einen zu dem anderen der beiden Bereiche strömen kann, unbeachtlich etwaiger zwischengeschalteter Bereiche, Bauteile oder benötigter Fördermittel.

Alle etwaigen Druckangaben erfolgen in bar als Absolutdruckeinheiten, abgekürzt bara, oder als Überdruckeinheiten, abgekürzt barg, wenn im jeweiligen Einzelzusammenhang nichts anderes angegeben wird.

Der Erfindung liegt die Erkenntnis zugrunde, dass es vorteilhaft ist, in einem Verfahren zur parallelen Erzeugung von Methanol und Ammoniak möglichst alle anfallenden Stoffströme bevorzugt stofflich zu nutzen. Dies gelingt gemäß Anspruch 1 durch folgende Maßnahmen:
(1) Mindestens eines Teils des Methanolsynthese-Spülstroms wird in die Sorptionsvorrichtung eingeleitet, anstatt ihn beispielsweise thermisch als Heizgas zu nutzen. In der Sorptionsvorrichtung wird der im Methanolsynthese-Spülstrom enthaltene Anteil an Kohlendioxid abgetrennt. Dies erfolgt bevorzugt gemeinsam mit dem aus der CO-Konvertierungsanlage ausgeleiteten und ebenfalls in die Sorptionsvorrichtung eingeleiteten, konvertierten Synthesegasstrom. Hierdurch kann - nach der Auftrennung des aus der Sorptionsvorrichtung ausgeleiteten, entsäuerten Synthesegasstroms in der Flüssigstickstoffwaschstufe - weiterer Wasserstoff gewonnen und der Ammoniaksynthese zugeführt werden.
(2) Es werden einer oder mehrerer Gasströme in den Methanolsynthesereaktor eingeleitet, die ausgewählt sind aus der folgenden Gruppe:
   (2.1) ein Teil des konvertierten Synthesegasstroms aus Verfahrensschritt (h)
   (2.2) ein Teil des entsäuerten Synthesegasstroms aus Verfahrensschritt (i)
   (2.3) mindestens ein Teil des zweiten Restgasstroms aus Verfahrensschritt (j2)

Auf diese Weise ist es möglich, das für die Methanolsynthese gewünschte, stöchiometrische H₂/CO-Verhältnis von ≥ 2, beispielsweise von 2,1 einzustellen, ohne dass hierfür ein Teil des Reinwasserstoffs verbraucht werden muss, der somit uneingeschränkt der Ammoniaksynthese zur Verfügung steht.

Die unter (1) und (2) genannten Maßnahmen wirken ferner in vorteilhafter Weise zusammen, da durch deren Kombination insgesamt mehr Wasserstoff mit geringerem Energieaufwand für die Ammoniaksynthese einerseits und für die Einstellung der Stöchiometriezahl für die Methanolsynthese andererseits bereitgestellt werden kann. Die beiden Teilverfahren Methanolsynthese und Ammoniaksynthese wirken vorteilhaft und in stärkerer Weise synergistisch zusammen, als dies aus der Beschreibung entsprechender Kombinationsverfahren im Stand der Technik bekannt war. Gleichzeitig werden aber durch die Führung eines Stoffstroms von der Methanolsynthese zur Ammoniaksynthese und durch die Führung eines oder mehrerer Stoffstroms von der Ammoniaksynthese zur Methanolsynthese, bzw. der der Ammoniaksynthese vorgelagerten Synthesegas-Konditionierung, die beiden Teilverfahren entkoppelt, so dass Fluktuationen in einem der Teilverfahren bis zu einem gewissen Grad durch die Änderung dieser Stoffströme ausgeglichen werden können.

Bei dem erfindungsgemäßen Verfahren kann der gesamte benötigte Wasserstoff sowohl für die Einstellung des stöchiometrischen Verhältnisses im Methanolsynthese-Einsatzgas als auch im Ammoniak-Synthesegas durch eine CO-Shift-Einheit erzeugt werden. Die Weiterleitung eines Teils des Rohwasserstoffs, der nach der CO₂- und Methan-Entfernung noch CO enthält, an die Methanolsynthese kann zur Einstellung des stöchiometrischen Verhältnisses in der Methanolsynthese genutzt werden, ohne dass das verbleibende CO an Brenngas verloren geht, wie es in Wasserstoffrückgewinnungsanlagen gemäß Stand der Technik geschieht.

Die erfindungsgemäße Zuführung eines wasserstoffreichen Stroms aus der Flüssigstickstoffwäsche zur Methanolsynthese, bei der nur Methan entfernt wird, nutzt das nicht umgewandelte CO in diesem Stoffstrom maximal aus, da dieses CO an der Reaktion in der Methanolsynthese teilnimmt und somit stofflich genutzt wird.

Insbesondere die Anpassung der Stöchiometriezahl in der Methanolsynthese durch die Zuführung von wasserstoffreichem, CO-haltigem Gas, bei dem in einem ersten Waschschritt der Flüssigstickstoffwäsche nur Methan entfernt wurde, kann mit sehr geringen Wasserstoffverlusten bewerkstelligt werden. In derselben Flüssigstickstoffwäsche oder in einer weiteren Trennkolonne wird in einem zweiten Waschschritt ein zweiter, wasserstoffreicher Gasstrom mit sehr wenig, typischerweise < 20 Vol.ppm CO erzeugt, der als Einsatzstrom für die Ammoniaksynthese verwendet wird.

Vorteilhaft bei der erfindungsgemäßen Verfahrensführung ist es ferner, dass aus dem zweiten Rohsynthesegasteilstrom ein entsäuerter Synthesegasstrom erhalten wird, der in der Sorptionsvorrichtung bis in den ppm-Bereich hinein von Kohlendioxid befreit und zusätzlich getrocknet wird. Hierdurch müssen Trocknungs- und Kohlendioxid-Feinentfernungsvorrichtungen, die in einem Beispiel der Flüssigstickstoffwäsche vorgeschaltet sind, weniger groß ausgestaltet werden.

Weiterhin ist es vorteilhaft, dass der aus der Flüssigstickstoffwäsche erhaltene Ammoniaksynthesefeedstrom tiefkalt anfällt und vollständig oder als Teilstrom vorteilhaft in der Sorptionsanlage als Kältemittel zur Abkühlung des konvertierten Synthesegasstroms vor Einleiten in die Sorptionsvorrichtung genutzt werden kann. Hierdurch wird Energie für die Kälteerzeugung eingespart und die Energieeffizienz des Gesamtverfahrens weiter verbessert. Der hierdurch vorgewärmte Ammoniaksynthesefeedstrom wird sodann der Ammoniaksynthese zugeführt.

### Besondere Ausgestaltungen der Erfindung

In einem zweiten Aspekt der Erfindung ist das erfindungsgemäße Verfahren dadurch gekennzeichnet, dass die Synthesegaserzeugungsanlage
(b2) eine Autothermreformierungsstufe (ATR) oder
(b3) eine Partialoxidationsstufe (POX) oder
(b4) eine Kombination der Stufen (b2) bis (b3) miteinander oder mit einer mittels Brennern und/oder heißen Gasen beheizte Dampfreformierungsstufe
umfasst und dass der erzeugte Rohsynthesegasstrom eine Stöchiometriezahl kleiner als 2 aufweist. Bei diesen Ausgestaltungen der Synthesegaserzeugungsanlage werden mit der Erfindung besondere Vorteile erzielt, da der erzeugte Rohsynthesegasstrom eine Stöchiometriezahl kleiner als 2, in einem Beispiel kleiner oder gleich 1,8, in einem weiteren Beispiel kleiner oder gleich 1,7 aufweist. Die erörterte stoffliche Nutzung des Methanolsynthese-Spülstroms und des bzw. der aus dem zweiten Rohsynthesegasteilstrom gewonnenen Gasströme, die der Methanolsynthese zugeführt werden, führt hierbei in besonders effektiver Weise zu einer Verbesserung des Wasserstoffhaushalts des Gesamtverfahrens. Gleichzeitig bestehen mit diesen Ausgestaltungen der Synthesegaserzeugungsanlage wirtschaftliche Vorteile, da sie technisch und ökonomisch weniger aufwendig sind als beispielsweise eine Ausgestaltung mit Dampfreformierung. Vorteile ergeben sich dagegen auch bei einer Kombination der Stufen (b2) bis (b3) miteinander oder mit einer mittels Brennern und/oder heißen Gasen beheizte Dampfreformierungsstufe, wenn der mit dieser Kombination erzeugte Rohsynthesegasstrom eine Stöchiometriezahl kleiner als 2, in einem Beispiel kleiner oder gleich 1,8, in einem weiteren Beispiel kleiner oder gleich 1,7 aufweist.

In einem dritten Aspekt der Erfindung ist das erfindungsgemäße Verfahren dadurch gekennzeichnet, dass der erzeugte Rohsynthesegasstrom einen Druck von 40 bara oder höher, bevorzugt 50 bara oder höher, meist bevorzugt 60 bara oder höher aufweist. Vorteilhaft ist es hierbei, dass der Verdichtungsaufwand für den ersten Rohsynthesegasteilstrom, der zur Methanolsynthese geführt wird, und den zweiten Rohsynthesegasteilstrom, der nach weiterer Konditionierung zur Ammoniaksynthese geführt wird, signifikant reduziert wird. Beides sind Hochdrucksyntheseverfahren; der Synthesedruck bei der Methanolsynthese beträgt in einem Beispiel zwischen 50 und 100 bara und der Synthesedruck bei der Ammoniaksynthese beträgt in einem Beispiel zwischen 250 und 350 bara. Insbesondere die mit dem zweiten Aspekt der Erfindung verbundenen Ausgestaltungen der Synthesegaserzeugungsanlage sind im Zusammenhang mit dem dritten Aspekt der Erfindung vorteilhaft einsetzbar, da sowohl Autothermreformierungsstufen als auch Partialoxidationsstufen üblicherweise bei erhöhten Drücken deutlich über Umgebungsdruck betrieben werden. Typische Druckbereiche liegen für Autothermreformierungsstufen bei 40 bis 60 bara und für Partialoxidationsstufen bei 40 bis 80 bara.

Bei der Implementierung des dritten Aspekts der Erfindung wird der Druck bereits im Syngas-Erzeugungsteil erhöht, was die gesamte Kompressionsenergie reduziert und auch den Einsatz von physikalischen CO₂-Entfernungstechniken begünstigt.

In einem vierten Aspekt der Erfindung ist das erfindungsgemäße Verfahren dadurch gekennzeichnet, dass die Sorptionsvorrichtung mittels eines physikalischen Absorptionsverfahrens arbeitet und dass sich die Sorptionsvorrichtung auf demselben Druckniveau befindet wie die Synthesegaserzeugungsanlage. Auch hier ist ein gegenüber Umgebungsdruck erhöhtes Druckniveau der Synthesegaserzeugungsanlage vorteilhaft, da die Löslichkeit des oder der aus dem konvertierten Synthesegasstrom abzutrennenden, sauren Gasbestandteile in dem Absorbens mit steigendem Druck wächst und sie somit effektiver und mit einer geringeren Absorbensmenge abgetrennt werden können.

In einem fünften Aspekt der Erfindung ist das erfindungsgemäße Verfahren dadurch gekennzeichnet, dass die Sorptionsvorrichtung mittels Gaswäsche mit kaltem Methanol arbeitet und dass ein kohlendioxidreicher Strom aus der Sorptionsvorrichtung ausgeleitet wird, dessen CO₂-Gehalt mindestens 98 Vol.-%, bevorzugt mindestens 99 Vol.-%, meist bevorzugt mindestens 99,5 Vol.-% beträgt. Es handelt sich hierbei um ein bewährtes physikalisches Absorptionsverfahren, das sich durch hohe Löslichkeitsunterscheide zwischen den Zielkomponenten, beispielsweise Wasserstoff und Kohlenmonoxid, und den sauren Gasbestandteilen, beispielsweise Kohlendioxid, als Störkomponenten auszeichnet, so dass die genannten, hohen CO₂-Gehalte mit geringem Aufwand erreicht werden können. Vorteilhaft gegenüber dem häufig zur CO₂-Entfernung aus Ammoniak-Synthesegas benutzten, chemisch-absorptiven Waschverfahren mit Aminen, beispielsweise mit Methyldiethanolamin (MDEA) ist es dabei, dass die Regenerierung des physikalischen Waschmittels Methanol erheblich leichter möglich ist, wodurch sich der Dampfverbrauch für die Regenerierung verringert und sich die Energiebilanz des Gesamtverfahrens gegenüber aus dem Stand der Technik bekannten Kombinationsverfahren weiter verbessert. So betragen typische Werte für benötigte Reboiler-Leistungen in Waschmittel-Regenerierungsanlagen für die Aminwäsche ca. 225 MW, bei der Methanolwäsche (Rectisol-Verfahren) dagegen nur ca. 25 MW.

In einem sechsten Aspekt der Erfindung ist das erfindungsgemäße Verfahren dadurch gekennzeichnet, dass der aus der Sorptionsvorrichtung ausgeleitete, kohlendioxidreiche Strom einem CO₂-Abscheidungs- und -Speicherungsverfahren (CCS) und/oder einem Verfahren zur stofflichen Nutzung von Kohlendioxid zugeführt wird. Aufgrund der mit geringem Aufwand erzielbaren, hohen CO₂-Gehalte wird eine effiziente Weiterverarbeitung des Sauergasstroms ermöglicht wird, da er sich direkt oder nach wenig aufwendiger Feinreinigung beispielsweise als Chemierohstoff oder für die CO₂-Sequestrierung eignet.

In einem siebten Aspekt der Erfindung ist das erfindungsgemäße Verfahren dadurch gekennzeichnet, dass der eine oder die mehreren Gasströme, die in den Methanolsynthesereaktor eingeleitet werden, bezogen auf ihren Stoffmengenstrom so eingestellt werden, dass die Stöchiometriezahl der Summe der in den Methanolsynthesereaktor eintretenden Feedströme mindestens 2 oder größer, bevorzugt mindestens 2,1 oder größer ist. Es ist wichtig, beim Zurückführen und Einleiten des oder der aus dem konvertierten Synthesegasstrom gewonnenen, wasserstoffhaltigen Ströme in den Methanolsynthesereaktor den bzw. die Mengenströme so zu wählen, dass die Stöchiometriezahl unter Berücksichtigung aller in den Methanolsynthesereaktor eintretenden Stoffströme in dem genannten Bereich von mindestens 2 oder größer, bevorzugt mindestens 2,1 oder größer liegt.

In einem achten Aspekt der Erfindung ist das erfindungsgemäße Verfahren dadurch gekennzeichnet, dass mehrere Gasströme gleichzeitig in den Methanolsynthesereaktor eingeleitet werden, wobei die mehreren Gasströme umfassen: (m3) Mindestens einen Teil des zweiten Restgasstroms aus Verfahrensschritt (j2) und zusätzlich einen oder mehrere weitere Gasströme, die ausgewählt sind aus der folgenden Gruppe:
(m1) ein Teil des konvertierten Synthesegasstroms aus Verfahrensschritt (h)
(m2) ein Teil des entsäuerten Synthesegasstroms aus Verfahrensschritt (i)

Vorteilhaft ist es dabei, dass die Verwendung mehrerer Gasströme zur Einstellung der gewünschten Stöchiometriezahl für die Methanolsynthese eine erhöhte Flexibilität erbringt, so dass zeitliche Veränderungen oder Fluktuationen eines der Gasströme durch eine Anpassung eines anderen der verwendeten Gasströme ausgeglichen werden können. Dies kann vorteilhaft bei instationären Anlagen- oder Verfahrenszuständen sein, beispielsweise bei der Inbetriebnahme oder der Außerbetriebnahme des Verfahrens oder der Anlage. Wie im Zusammenhang mit dem siebten Aspekt der Erfindung erläutert, ist es wichtig, dass beim Zurückführen und Einleiten der mehreren wasserstoffhaltigen Ströme in den Methanolsynthesereaktor die Mengenströme so zu wählen, dass die Stöchiometriezahl unter Berücksichtigung aller in den Methanolsynthesereaktor eintretenden Stoffströme in dem genannten Bereich von mindestens 2 oder größer, bevorzugt mindestens 2,1 oder größer liegt.

In einem neunten Aspekt der Erfindung ist die erfindungsgemäße Anlage dadurch gekennzeichnet, dass die Synthesegaserzeugungsanlage umfasst:
(b2) eine Autothermreformierungsstufe (ATR) oder
(b3) eine Partialoxidationsstufe (POX) oder
(b4) eine Kombination der Stufen (b2) bis (b3)

Die mit diesem Aspekt verbundenen technischen Effekte und Vorteile entsprechen denen, die im Zusammenhang mit dem zweiten Aspekt der Erfindung erörtert wurden.

In einem zehnten Aspekt der Erfindung ist die erfindungsgemäße Anlage dadurch gekennzeichnet, dass mindestens eine Verdichtungsstufe umfasst wird, die es ermöglicht, dass der erzeugte Rohsynthesegasstrom einen Druck von 40 bara oder höher, bevorzugt 50 bara oder höher, meist bevorzugt 60 bara oder höher aufweist. Die mit diesem Aspekt verbundenen technischen Effekte und Vorteile entsprechen denen, die im Zusammenhang mit dem dritten Aspekt der Erfindung erörtert wurden.

In einem elften Aspekt der Erfindung ist die erfindungsgemäße Anlage dadurch gekennzeichnet, dass die Sorptionsvorrichtung mittels eines physikalischen Absorptionsverfahrens arbeitet und so ausgestaltet ist, dass sich die Sorptionsvorrichtung auf demselben Druckniveau befindet wie die Synthesegaserzeugungsanlage. Die mit diesem Aspekt verbundenen technischen Effekte und Vorteile entsprechen denen, die im Zusammenhang mit dem vierten und fünften Aspekt der Erfindung erörtert wurden.

In einem zwölften Aspekt der Erfindung ist die erfindungsgemäße Anlage dadurch gekennzeichnet, dass die Sorptionsvorrichtung mittels Gaswäsche mit kaltem Methanol arbeitet und so ausgestaltet ist, dass ein kohlendioxidreicher Strom aus der Sorptionsvorrichtung ausgeleitet wird, dessen CO₂-Gehalt mindestens 98 Vol.-%, bevorzugt mindestens 99 Vol.-%, meist bevorzugt mindestens 99,5 Vol.-% beträgt. Die mit diesem Aspekt verbundenen technischen Effekte und Vorteile entsprechen denen, die im Zusammenhang mit dem fünften Aspekt der Erfindung erörtert wurden.

In einem dreizehnten Aspekt der Erfindung ist die erfindungsgemäße Anlage dadurch gekennzeichnet, dass Mittel umfasst werden, die es ermöglichen, dass der aus der Sorptionsvorrichtung ausgeleitete, kohlendioxidreiche Strom einem CO₂-Abscheidungs- und - Speicherungsverfahren (CCS) und/oder einem Verfahren zur stofflichen Nutzung von Kohlendioxid zugeführt wird. Die mit diesem Aspekt verbundenen technischen Effekte und Vorteile entsprechen denen, die im Zusammenhang mit dem sechsten Aspekt der Erfindung erörtert wurden.

In einem vierzehnten Aspekt der Erfindung ist die erfindungsgemäße Anlage dadurch gekennzeichnet, dass Mittel umfasst werden, die es ermöglichen, dass der eine oder die mehreren Gasströme, die in den Methanolsynthesereaktor eingeleitet werden, bezogen auf ihren Stoffmengenstrom so eingestellt werden, dass die Stöchiometriezahl der Summe der in den Methanolsynthesereaktor eintretenden Feedströme mindestens 2 oder größer, bevorzugt mindestens 2,1 oder größer ist. Die mit diesem Aspekt verbundenen technischen Effekte und Vorteile entsprechen denen, die im Zusammenhang mit dem siebten Aspekt der Erfindung erörtert wurden.

### Ausführungs- und Zahlenbeispiele

Weiterbildungen, Vorteile und Anwendungsmöglichkeiten der Erfindung ergeben sich auch aus der nachfolgenden Beschreibung von Ausführungs- und Zahlenbeispielen und den Zeichnungen. Dabei bilden alle beschriebenen und/oder bildlich dargestellten Merkmale für sich oder in beliebiger Kombination die Erfindung, unabhängig von ihrer Zusammenfassung in den Ansprüchen oder deren Rückbeziehung.

Es zeigt die einzige Figur:

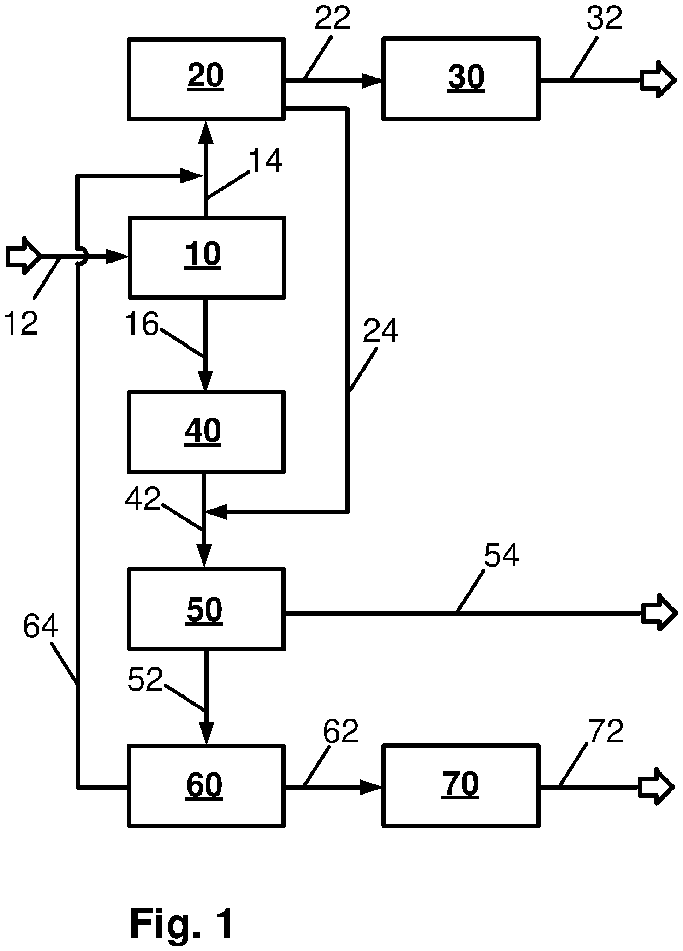

- Fig. 1: eine schematische Darstellung des Verfahrens bzw. der Anlage gemäß einer Ausführungsform der Erfindung.

In der in Fig. 1 dargestellten Ausgestaltung eines Verfahrens bzw. einer Anlage gemäß der Erfindung wird über Leitung 12 ein Kohlenwasserstoffe enthaltender Einsatzstrom, beispielsweise Erdgas, in einem bevorzugten Beispiel Erdgas mit einem Methangehalt von mindestens 80 Vol.-%, einer Synthesegaserzeugungsanlage 10 zugeführt, die in dieser Ausgestaltung einen Autothermreformer (ATR) umfasst und in einem Beispiel bei einem Druck von 60 bara betrieben wird.

In der Synthesegaserzeugungsanlage 10 erfolgt ein mindestens teilweises Umsetzen des Kohlenwasserstoffe enthaltenden Einsatzstroms unter Synthesegaserzeugungsbedingungen zu einem Wasserstoff (H₂), Kohlenmonoxid (CO) und Inertkomponenten wie Methan (CH₄) enthaltenden Rohsynthesegasstrom, der in einen ersten Rohsynthesegasteilstrom und in einen zweiten Rohsynthesegasteilstrom aufgeteilt wird.

Über Leitung 14 wird der erste Rohsynthesegasteilstrom aus der Synthesegaserzeugungsanlage ausgeleitet und einem Methanolsynthesereaktor 20 zugeführt, in dem ein mindestens teilweises Umsetzen des ersten Rohsynthesegasteilstroms unter Methanolsynthesebedingungen folgt. Über Leitung 22 wird ein Methanol enthaltender erster Reaktorproduktstrom aus dem Methanolsynthesereaktor 20 ausgeleitet, unter seinen Taupunkt abgekühlt und in einer bildlich nicht separat gezeigten Phasentrennvorrichtung in einen ersten Flüssigproduktstrom und einen ersten Restgasstrom aufgetrennt. Der erste Flüssigproduktstrom wird über Leitung 22 als Rohmethanolproduktstrom einer Methanolaufarbeitungsvorrichtung 30 zugeführt, die in einem Beispiel als Destillation, bevorzugt mehrstufige Destillation ausgestaltet ist. Aus der Methanolaufarbeitungsvorrichtung 30 wird über Leitung 32 ein Reinmethanolstrom ausgeleitet und der weiteren Verarbeitung oder Verwendung zugeführt.

Der erste Restgasstrom enthält nicht umgesetzte Synthesegasbestandteile, also Wasserstoffe und Kohlenoxide, insbesondere Kohlenmonoxid und Kohlendioxid, und Inertkomponenten, beispielsweise nicht in der Synthesegaserzeugungsanlage umgesetztes Methan und/oder Edelgase wie beispielsweise Argon. Der erste Restgasstrom wird in einen Methanolsynthese-Spülstrom und in einen Recyclestrom aufgeteilt, wobei der Recyclestrom zum Methanolsynthesereaktor zurückgeführt wird (bildlich nicht separat gezeigt) und der Methanolsynthese-Spülstrom über Leitung 24 aus dem Methanolsynthesereaktor ausgeleitet wird.

Über Leitung 16 wird der zweite Rohsynthesegasteilstrom aus der Synthesegaserzeugungsanlage ausgeleitet und eine CO-Konvertierungsanlage 40 eingeleitet, die mindestens eine CO-Konvertierungsstufe umfasst. In der CO-Konvertierungsstufe erfolgt unter Zugabe von Wasserdampf (bildlich nicht separat gezeigt) ein Umsetzen des im zweiten Rohsynthesegasteilstrom enthaltenen Kohlenmonoxids unter CO-Konvertierungsbedingungen zu einem konvertierten Synthesegasstrom, dessen Gehalt an Wasserstoff und Kohlendioxid gegenüber dem zweiten Rohsynthesegasteilstrom erhöht wurde. Der konvertierte Synthesegasstrom wird über Leitung 42 aus der CO-Konvertierungsanlage 40 ausgeleitet.

Über Leitung 42 wird der konvertierte Synthesegasstrom in eine Sorptionsvorrichtung 50 zur Entfernung saurer Gasbestandteile, insbesondere Kohlendioxid, mittels eines physikalischen oder chemischen Sorptionsverfahrens eingeleitet. In einem Beispiel ist die Sorptionsvorrichtung 50 zur Durchführung einer Gaswäsche mit dem physikalischen Absorbens Methanol ausgestaltet (Rectisol-Verfahren). Hierbei wird ein entsäuerter Synthesegasstrom erhalten, der über Leitung 52 aus der Sorptionsvorrichtung 50 ausgeleitet wird. Ferner wird ein saure Gasbestandteile enthaltender Sauergasstrom erhalten, der über Leitung 54 ausgeleitet wird. In einem Beispiel wird die Sorptionsvorrichtung 50 so ausgestaltet und betrieben, dass über Leitung 54 ein trockener, kohlendioxidreicher Strom aus der Sorptionsvorrichtung ausgeleitet wird, dessen CO₂-Gehalt mindestens 98 Vol.-%, bevorzugt mindestens 99 Vol.-%, meist bevorzugt mindestens 99,5 Vol.-% beträgt. Dies ermöglicht, dass der aus der Sorptionsvorrichtung ausgeleitete, kohlendioxidreiche Strom bevorzugt direkt, also ohne weitere Konditionierungs- oder Reinigungsschritt, einem CO₂-Abscheidungs- und -Speicherungsverfahren (CCS) und/oder einem Verfahren zur stofflichen Nutzung von Kohlendioxid zugeführt wird.

Über Leitung 52 wird der entsäuerte Synthesegasstrom in eine Flüssigstickstoffwaschstufe 60 eingeleitet. In einem Beispiel wird der entsäuerte Synthesegasstrom vor dem Zuführen in Flüssigstickstoffwaschstufe 60 einer oder mehreren Trocknungs- und Kohlendioxid-Feinentfernungsvorrichtungen (bildlich nicht separat gezeigt) zugeführt, um Spuren an Wasser und/oder Kohlendioxid zu entfernen, die ansonsten in der Flüssigstickstoffwaschstufe ausfrieren und dort zu Verstopfungen führen können. Vorteilhaft ist es dabei, dass aus dem zweiten Rohsynthesegasteilstrom ein entsäuerter Synthesegasstrom erhalten wird, der in der Sorptionsvorrichtung bis in den ppm-Bereich hinein von Kohlendioxid befreit und zusätzlich getrocknet wird. Hierdurch müssen Trocknungs- und Kohlendioxid-Feinentfernungsvorrichtungen, die in diesem Beispiel der Flüssigstickstoffwäsche vorgeschaltet sind, weniger groß ausgestaltet werden.

In der Flüssigstickstoffwaschstufe 60 erfolgt ein Auftrennen, beispielsweise ein mehrstufiges Auftrennen, des entsäuerten Synthesegasstroms in der Flüssigstickstoffwaschstufe 60 in folgende Teilströme:
(60.1) einen Wasserstoff und Stickstoff als Hauptbestandteile und Kohlenmonoxid und Inertkomponenten als Spurenbestandteile enthaltenden Ammoniaksynthesefeedstrom,
(60.2) einen Wasserstoff und Kohlenmonoxid als Hauptbestandteile und Inertkomponenten als Spurenbestandteile enthaltenden zweiten Restgasstrom,
(60.3) einen Inertkomponenten als Hauptbestandteil enthaltenden Inertgasstrom, der aus dem Verfahren ausgeleitet wird.

Der erhaltene Stoffstrom (60.1) wird über Leitung 62 als Ammoniaksynthesefeedstrom in einen Ammoniaksynthesereaktor 70 eingeleitet. Durch entsprechende Ausgestaltung der Flüssigstickstoffwaschstufe 60 bzw. ihres Betriebs wird gewährleitet, dass die in dem Ammoniaksynthesefeedstrom enthaltenen Spurenanteile von Kohlenmonoxid und Inertkomponenten die nachfolgende Ammoniaksynthese nicht nachteilig beeinflussen. Ferner wird gewährleitet, dass der Ammoniaksynthesefeedstrom ein Wasserstoff/Stickstoff-Gemisch gewünschter Zusammensetzung enthält, beispielsweise mit einem molaren ein Wasserstoff/Stickstoff-Verhältnis von 3 gemäß der Stöchiometrie der Ammoniaksynthesereaktion.

Der erhaltene Stoffstrom (60.3), der Inertkomponenten, insbesondere Methan als Hauptbestandteil enthält, wird nach Ausleiten aus dem Verfahren beispielsweise aufgrund seines Heizwertes als Heizgas verwendet. Alternativ kann er als Teil des Kohlenwasserstoffe enthaltender Einsatzstrom zu der Synthesegaserzeugungsanlage 10 zurückgeführt werden. Falls der Strom (60.3) signifikante Anteile an Komponenten wie beispielsweise Argon enthält, die nicht in der Synthesegaserzeugungsanlage umgesetzt werden können, empfiehlt es sich, nur einen Teil des Stroms (60.3) zu der Synthesegaserzeugungsanlage zurückzuführen, um eine Akkumulation dieser Stoffe zu vermeiden.

In dem Ammoniaksynthesereaktor 70 erfolgt ein mindestens teilweises Umsetzen des Ammoniaksynthesefeedstroms unter Ammoniaksynthesebedingungen. Sodann wird über Leitung 72 ein Ammoniakproduktstroms aus dem Ammoniaksynthesereaktor 70 ausgeleitet und der weiteren Verwendung oder Verarbeitung zugeführt.

Erfindungsgemäß wird über Leitung 24 mindestens eines Teils des Methanolsynthese-Spülstroms in die Sorptionsvorrichtung 50 eingeleitet. Das Einleiten kann dabei direkt in die Sorptionsvorrichtung und/oder in die in die Sorptionsvorrichtung einmündende Leitung 42 erfolgen. In der Sorptionsvorrichtung wird auch aus dem Methanolsynthese-Spülstrom der Kohlendioxid-Anteil abgetrennt, so dass die verbliebenen Anteile an Kohlenmonoxid und Wasserstoff besser in den nachfolgenden Verfahrensschritten bzw. Anlagenteilen genutzt werden können.

Ferner wird erfindungsgemäß über Leitung 64 der Wasserstoff und Kohlenmonoxid als Hauptbestandteile enthaltende zweite Restgasstrom (60.2) zum Methanolsynthesereaktor 20 zurückgeführt, so dass diese Hauptbestandteile in der Methanolsynthese stofflich genutzt werden können. Alternativ oder zusätzlich können auch
- ein Teil des konvertierten Synthesegasstroms aus der CO-Konvertierungsstufe oder stromabwärts davon und/oder
- ein Teil des entsäuerten Synthesegasstroms aus der Sorptionsvorrichtung oder stromabwärts davon
zum Methanolsynthesereaktor 20 zurückgeführt werden (beides bildlich nicht separat gezeigt). Auch Mischungen dieser drei potentiellen Rückführströme sind möglich, so dass eine noch größere Flexibilität hinsichtlich der Einstellung von Rückführströme zum Methanolsynthesereaktor besteht.

Durch die Rückführung eines oder mehrerer der genannten Stoffströme können die in ihnen erhaltenen Anteile an Wasserstoff und Kohlenmonoxid stofflich im Methanolsynthesereaktor zur Erzeugung zusätzlichen Methanols genutzt werden. Ferner kann hierdurch die gewünschte Stöchiometriezahl im Methanolsynthesereaktor eingestellt werden, ohne dass verfahrensfremder Wasserstoff importiert werden muss oder der Wasserstoff einem Reinwasserstoffstrom entnommen werden muss. Ein solcher Reinwasserstoffstrom steht innerhalb des erfindungsgemäßen Verfahrens ohnehin nicht ohne weiteres zur Verfügung, da der Strom (60.1) bereits den stöchiometrischen Anteil an Stickstoff enthält. Stickstoff ist im Sinne der Methanolsynthese eine Inertkomponente und daher dort unerwünscht.

Die Erhöhung des Drucks in der Synthesegaserzeugungsanlage auf 60 bara wirkt sich positiv auf die Gesamtwirtschaftlichkeit des Verfahrens aus, da sie zu einer Verringerung der für die Methanolsynthese benötigten Kompressionsenergie beiträgt. Ferner resultiert aus der Druckerhöhung auch eine gegenüber niedrigeren Drücken verbesserten Absorption von Kohlendioxid in dem physikalischen Waschmittel Methanol in der nach dem Rectisol-Verfahren ausgestalteten Sorptionsvorrichtung.

Die Weiterleitung von Spülgas aus dem Methanolsynthesereaktor zur CO₂-Entfernung in der Sorptionsvorrichtung und nachfolgend zur kryogenen Entfernung von Methan und die anschließende Weiterleitung des gereinigten Rohwasserstoffs und des in ihm verbleibenden CO-Anteils zur Methanolsynthese verbessert die Effizienz des Gesamtprozesses, ohne dass zusätzliche Reinigungsvorrichtungen wie eine Druckwechseladsorption (PSA) oder Membrananlagen erforderlich sind.

### Bezugszeichenliste

- [10]: Synthesegaserzeugungsanlage
- [12]: Leitung
- [14]: Leitung
- [16]: Leitung
- [20]: Methanolsynthesereaktor
- [22]: Leitung
- [24]: Leitung
- [30]: Methanolaufarbeitungsvorrichtung (Methanoldestillation)
- [40]: CO-Konvertierungsanlage
- [42]: Leitung
- [50]: Sorptionsvorrichtung (Rectisol)
- [52]: Leitung
- [54]: Leitung
- [60]: Flüssigstickstoffwaschstufe
- [62]: Leitung
- [64]: Leitung
- [70]: Ammoniaksynthesereaktor
- [72]: Leitung

## Patentansprüche

1. Verfahren zum Herstellen von Methanol und Ammoniak aus einem Kohlenwasserstoffe, bevorzugt Methan, meist bevorzugt Erdgas mit einem Methangehalt von mindestens 80 Vol.-%, enthaltenden Einsatzstrom, umfassend folgende Schritte:
(a) Bereitstellen des Kohlenwasserstoffe enthaltenden Einsatzstroms,
(b) Zuführen des Kohlenwasserstoffe enthaltenden Einsatzstroms zu einer Synthesegaserzeugungsanlage, umfassend
(b1) eine mittels Brennern und/oder heißen Gasen beheizte Dampfreformierungsstufe oder
(b2) eine Autothermreformierungsstufe (ATR) oder
(b3) eine Partialoxidationsstufe (POX) oder
(b4) eine Kombination von mindestens zwei der Stufen (b1) bis (b3),
(c) mindestens teilweises Umsetzen des Kohlenwasserstoffe enthaltenden Einsatzstroms in der Synthesegaserzeugungsanlage unter Synthesegaserzeugungsbedingungen zu einem Wasserstoff (H₂), Kohlenmonoxid (CO) und Inertkomponenten wie Methan (CH₄) enthaltenden Rohsynthesegasstrom,
(d) Ausleiten eines Rohsynthesegasstroms aus der Synthesegaserzeugungsanlage und Aufteilen des Rohsynthesegasstroms in einen ersten Rohsynthesegasteilstrom und in einen zweiten Rohsynthesegasteilstrom,
(e) Einleiten mindestens eines Teils des ersten Rohsynthesegasteilstroms in einen Methanolsynthesereaktor, mindestens teilweises Umsetzen des ersten Rohsynthesegasteilstroms in dem Methanolsynthesereaktor unter Methanolsynthesebedingungen,
(f) Ausleiten eines Methanol enthaltenden ersten Reaktorproduktstroms aus dem Methanolsynthesereaktor, Abkühlen des ersten Reaktorproduktstroms unter seinen Taupunkt und Auftrennen des abgekühlten ersten Reaktorproduktstroms in einer Phasentrennvorrichtung in einen ersten Flüssigproduktstrom und einen ersten Restgasstrom, der nicht umgesetzte Synthesegasbestandteile und Inertkomponenten enthält, Ausleiten des ersten Flüssigproduktstroms aus dem Verfahren als Rohmethanolproduktstrom,
(g) Aufteilen des ersten Restgasstroms in einen Methanolsynthese-Spülstrom und in einen Recyclestrom, der zum Methanolsynthesereaktor zurückgeführt wird,
(h) Einleiten mindestens eines Teils des zweiten Rohsynthesegasteilstroms in eine CO-Konvertierungsanlage, umfassend mindestens eine CO-Konvertierungsstufe, Umsetzen des in die CO-Konvertierungsanlage eingeleiteten Teils des zweiten Rohsynthesegasteilstroms unter CO-Konvertierungsbedingungen zu einem konvertierten Synthesegasstrom, Ausleiten des konvertierten Synthesegasstroms,
(i) Einleiten des konvertierten Synthesegasstroms in eine Sorptionsvorrichtung zur Entfernung saurer Gasbestandteile, insbesondere Kohlendioxid, mittels eines physikalischen oder chemischen Sorptionsverfahrens, Ausleiten eines entsäuerten Synthesegasstroms und eines saure Gasbestandteile enthaltenden Sauergasstroms aus der Sorptionsvorrichtung, Ausleiten des Sauergasstroms aus dem Verfahren,
(j) Einleiten mindestens eines Teils des entsäuerten Synthesegasstroms in eine Flüssigstickstoffwaschstufe, Auftrennen des entsäuerten Synthesegasstroms in der Flüssigstickstoffwaschstufe in folgende Teilströme:
(j1) einen Wasserstoff und Stickstoff als Hauptbestandteile und Kohlenmonoxid und Inertkomponenten als Spurenbestandteile enthaltenden Ammoniaksynthesefeedstrom,
(j2) einen Wasserstoff und Kohlenmonoxid als Hauptbestandteile und Inertkomponenten als Spurenbestandteile enthaltenden zweiten Restgasstrom,
(j3) einen Inertkomponenten als Hauptbestandteil enthaltenden Inertgasstrom, der aus dem Verfahren ausgeleitet wird,
(k) Einleiten des Ammoniaksynthesefeedstroms in einen Ammoniaksynthesereaktor, mindestens teilweises Umsetzen des Ammoniaksynthesefeedstroms in dem Ammoniaksynthesereaktor unter Ammoniaksynthesebedingungen, Ausleiten eines Ammoniakproduktstroms aus dem Ammoniaksynthesereaktor,
(I) Einleiten mindestens eines Teils des Methanolsynthese-Spülstroms in die Sorptionsvorrichtung,
(m) Einleiten eines oder mehrerer Gasströme in den Methanolsynthesereaktor, die ausgewählt sind aus der folgenden Gruppe:
(m1) ein Teil des konvertierten Synthesegasstroms aus Verfahrensschritt (h)
(m2) ein Teil des entsäuerten Synthesegasstroms aus Verfahrensschritt (i)
(m3) mindestens ein Teil des zweiten Restgasstroms aus Verfahrensschritt (j2).

2. Verfahren nach Anspruch 1, **dadurch gekennzeichnet, dass** die Synthesegaserzeugungsanlage
(b2) eine Autothermreformierungsstufe (ATR) oder
(b3) eine Partialoxidationsstufe (POX) oder
(b4) eine Kombination der Stufen (b2) bis (b3) miteinander oder mit einer mittels Brennern und/oder heißen Gasen beheizte Dampfreformierungsstufe
umfasst und dass der erzeugte Rohsynthesegasstrom eine Stöchiometriezahl kleiner als 2 aufweist.

3. Verfahren nach Anspruch 2, **dadurch gekennzeichnet, dass** der erzeugte Rohsynthesegasstrom einen Druck von 40 bara oder höher, bevorzugt 50 bara oder höher, meist bevorzugt 60 bara oder höher aufweist.

4. Verfahren nach Anspruch 3, **dadurch gekennzeichnet, dass** die Sorptionsvorrichtung mittels eines physikalischen Absorptionsverfahrens arbeitet und dass sich die Sorptionsvorrichtung auf demselben Druckniveau befindet wie die Synthesegaserzeugungsanlage.

5. Verfahren nach Anspruch 4, **dadurch gekennzeichnet, dass** die Sorptionsvorrichtung mittels Gaswäsche mit kaltem Methanol arbeitet und dass ein kohlendioxidreicher Strom aus der Sorptionsvorrichtung ausgeleitet wird, dessen CO₂-Gehalt mindestens 98 Vol.-%, bevorzugt mindestens 99 Vol.-%, meist bevorzugt mindestens 99,5 Vol.-% beträgt.

6. Verfahren nach Anspruch 5, **dadurch gekennzeichnet, dass** der aus der Sorptionsvorrichtung ausgeleitete, kohlendioxidreiche Strom einem CO₂-Abscheidungs- und -Speicherungsverfahren (CCS) und/oder einem Verfahren zur stofflichen Nutzung von Kohlendioxid zugeführt wird.

7. Verfahren nach einem der vorigen Ansprüche, **dadurch gekennzeichnet, dass** der eine oder die mehreren Gasströme, die in den Methanolsynthesereaktor eingeleitet werden, bezogen auf ihren Stoffmengenstrom so eingestellt werden, dass die Stöchiometriezahl der Summe der in den Methanolsynthesereaktor eintretenden Feedströme mindestens 2 oder größer, bevorzugt mindestens 2,1 oder größer ist.

8. Verfahren nach einem der vorigen Ansprüche, **dadurch gekennzeichnet, dass** mehrere Gasströme gleichzeitig in den Methanolsynthesereaktor eingeleitet werden, wobei die mehreren Gasströme umfassen: (m3) Mindestens einen Teil des zweiten Restgasstroms aus Verfahrensschritt (j2) und zusätzlich einen oder mehrere weitere Gasströme, die ausgewählt sind aus der folgenden Gruppe:
(m1) ein Teil des konvertierten Synthesegasstroms aus Verfahrensschritt (h)
(m2) ein Teil des entsäuerten Synthesegasstroms aus Verfahrensschritt (i)

9. Anlage zum Herstellen von Methanol und Ammoniak aus einem Kohlenwasserstoffe enthaltenden Einsatzstrom, umfassend folgende, miteinander in Fluidverbindung stehende Baugruppen und Bestandteile:
(a) Mittel zum Bereitstellen des Kohlenwasserstoffe enthaltenden Einsatzstroms,
(b) eine Synthesegaserzeugungsanlage, Mittel zum Zuführen des Kohlenwasserstoffe enthaltenden Einsatzstroms zu der Synthesegaserzeugungsanlage, wobei die Synthesegaserzeugungsanlage umfasst:
(b1) eine mittels Brennern und/oder heißen Gasen beheizte Dampfreformierungsstufe oder
(b2) eine Autothermreformierungsstufe (ATR) oder
(b3) eine Partialoxidationsstufe (POX) oder
(b4) eine Kombination von mindestens zwei der Stufen (b1) bis (b3),
(c) Mittel zum Ausleiten eines Rohsynthesegasstroms, enthaltend Wasserstoff (H₂), Kohlenmonoxid (CO) und Inertkomponenten wie Methan (CH₄), aus der Synthesegaserzeugungsanlage und Mittel zum Aufteilen des Rohsynthesegasstroms in einen ersten Rohsynthesegasteilstrom und in einen zweiten Rohsynthesegasteilstrom,
(d) einen Methanolsynthesereaktor, Mittel zum Einleiten mindestens eines Teils des ersten Rohsynthesegasteilstroms in einen Methanolsynthesereaktor,
(e) Mittel zum Ausleiten eines Methanol enthaltenden ersten Reaktorproduktstroms aus dem Methanolsynthesereaktor, Mittel zum Abkühlen des ersten Reaktorproduktstroms unter seinen Taupunkt, eine Phasentrennvorrichtung, Mittel zum Auftrennen des abgekühlten ersten Reaktorproduktstroms in der Phasentrennvorrichtung in einen ersten Flüssigproduktstrom und einen ersten Restgasstrom, der nicht umgesetzte Synthesegasbestandteile und Inertkomponenten enthält, Mittel zum Ausleiten des ersten Flüssigproduktstroms aus dem Verfahren als Rohmethanolproduktstrom,
(f) Mittel zum Aufteilen des ersten Restgasstroms in einen Methanolsynthese-Spülstrom und in einen Recyclestrom, der zum Methanolsynthesereaktor zurückgeführt wird,
(g) eine CO-Konvertierungsanlage, umfassend mindestens eine CO-Konvertierungsstufe, Mittel zum Einleiten mindestens eines Teils des zweiten Rohsynthesegasteilstroms in die CO-Konvertierungsanlage, Mittel zum Ausleiten eines konvertierten Synthesegasstroms aus der CO-Konvertierungsanlage,
(h) eine Sorptionsvorrichtung zur Entfernung saurer Gasbestandteile, insbesondere Kohlendioxid, mittels eines physikalischen oder chemischen Sorptionsverfahrens, Mittel zum Einleiten des konvertierten Synthesegasstroms in die Sorptionsvorrichtung, Mittel zum Ausleiten eines entsäuerten Synthesegasstroms und eines saure Gasbestandteile enthaltenden Sauergasstroms aus der Sorptionsvorrichtung, Mittel zum Ausleiten des Sauergasstroms aus dem Verfahren,
(i) eine Flüssigstickstoffwaschstufe, Mittel zum Einleiten mindestens eines Teils des entsäuerten Synthesegasstroms in die Flüssigstickstoffwaschstufe, Mittel zum Auftrennen des entsäuerten Synthesegasstroms in der Flüssigstickstoffwaschstufe in folgende Teilströme:
(i1) einen Wasserstoff und Stickstoff als Hauptbestandteile und Kohlenmonoxid und Inertkomponenten als Spurenbestandteile enthaltenden Ammoniaksynthesefeedstrom,
(i2) einen Wasserstoff und Kohlenmonoxid als Hauptbestandteile und Inertkomponenten als Spurenbestandteile enthaltenden zweiten Restgasstrom,
(i3) einen Inertkomponenten als Hauptbestandteil enthaltenden Inertgasstrom,
(j) einen Ammoniaksynthesereaktor, Mittel zum Einleiten des Ammoniaksynthesefeedstroms in den Ammoniaksynthesereaktor, Mittel zum Ausleiten eines Ammoniakproduktstroms aus dem Ammoniaksynthesereaktor,
(k) Mittel zum Einleiten eines oder mehrerer Gasströme in den Methanolsynthesereaktor, wobei die Gasströme ausgewählt sind aus der folgenden Gruppe:
(m1) ein Teil des konvertierten Synthesegasstroms aus Verfahrensschritt (h)
(m2) ein Teil des entsäuerten Synthesegasstroms aus Verfahrensschritt (i)
(m3) mindestens ein Teil des zweiten Restgasstroms aus Verfahrensschritt (j2).

10. Anlage nach Anspruch 9, **dadurch gekennzeichnet, dass** die Synthesegaserzeugungsanlage umfasst:
(b2) eine Autothermreformierungsstufe (ATR) oder
(b3) eine Partialoxidationsstufe (POX) oder
(b4) eine Kombination der Stufen (b2) bis (b3) miteinander oder mit einer mittels Brennern und/oder heißen Gasen beheizte Dampfreformierungsstufe.

11. Anlage nach Anspruch 10, **dadurch gekennzeichnet, dass** mindestens eine Verdichtungsstufe umfasst wird, die es ermöglicht, dass der erzeugte Rohsynthesegasstrom einen Druck von 40 bara oder höher, bevorzugt 50 bara oder höher, meist bevorzugt 60 bara oder höher aufweist.

12. Anlage nach Anspruch 11, **dadurch gekennzeichnet, dass** die Sorptionsvorrichtung mittels eines physikalischen Absorptionsverfahrens arbeitet und so ausgestaltet ist, dass sich die Sorptionsvorrichtung auf demselben Druckniveau befindet wie die Synthesegaserzeugungsanlage.

13. Anlage nach Anspruch 12, **dadurch gekennzeichnet, dass** die Sorptionsvorrichtung mittels Gaswäsche mit kaltem Methanol arbeitet und so ausgestaltet ist, dass ein kohlendioxidreicher Strom aus der Sorptionsvorrichtung ausgeleitet wird, dessen CO₂-Gehalt mindestens 98 Vol.-%, bevorzugt mindestens 99 Vol.-%, meist bevorzugt mindestens 99,5 Vol.-% beträgt.

14. Anlage nach Anspruch 13, **dadurch gekennzeichnet, dass** Mittel umfasst werden, die es ermöglichen, dass der aus der Sorptionsvorrichtung ausgeleitete, kohlendioxidreiche Strom einem CO₂-Abscheidungs- und -Speicherungsverfahren (CCS) und/oder einem Verfahren zur stofflichen Nutzung von Kohlendioxid zugeführt wird.

15. Anlage nach einem der vorigen Ansprüche, **dadurch gekennzeichnet, dass** Mittel umfasst werden, die es ermöglichen, dass der eine oder die mehreren Gasströme, die in den Methanolsynthesereaktor eingeleitet werden, bezogen auf ihren Stoffmengenstrom so eingestellt werden, dass die Stöchiometriezahl der Summe der in den Methanolsynthesereaktor eintretenden Feedströme mindestens 2 oder größer, bevorzugt mindestens 2,1 oder größer ist.

## Claims

1. Process for producing methanol and ammonia from an input stream containing hydrocarbons, preferably methane, most preferably natural gas having a methane content of at least 80% by volume, comprising the following steps of:
(a) providing the input stream containing hydrocarbons,
(b) supplying the input stream containing hydrocarbons to a synthesis gas production plant comprising
(b1) a steam reforming stage heated using burners and/or hot gases or
(b2) an autothermal reforming stage (ATR) or
(b3) a partial oxidation stage (POX) or
(b4) a combination of at least two of the stages (b1) to (b3),
(c) at least partially converting the input stream containing hydrocarbons in the synthesis gas production plant under synthesis gas production conditions to afford a raw synthesis gas stream containing hydrogen (H₂), carbon monoxide (CO) and inert components such as methane (CH₄),
(d) discharging a raw synthesis gas stream from the synthesis gas production plant and dividing the raw synthesis gas stream into a first raw synthesis gas substream and into a second raw synthesis gas substream,
(e) introducing at least a portion of the first raw synthesis gas substream into a methanol synthesis reactor, at least partially converting the first raw synthesis gas substream in the methanol synthesis reactor under methanol synthesis conditions,
(f) discharging a methanol-containing first reactor product stream from the methanol synthesis reactor, cooling the first reactor product stream to below its dew point and separating the cooled first reactor product stream in a phase separation apparatus into a first liquid product stream and a first residual gas stream containing unconverted synthesis gas constituents and inert components, discharging the first liquid product stream from the process as a raw methanol product stream,
(g) dividing the first residual gas stream into a methanol synthesis purge stream and into a recycle stream which is recycled to the methanol synthesis reactor,
(h) introducing at least a portion of the second raw synthesis gas substream into a CO conversion plant comprising at least one CO conversion stage, converting the portion of the second raw synthesis gas substream introduced into the CO conversion plant under CO conversion conditions to afford a converted synthesis gas stream, discharging the converted synthesis gas stream,
(i) introducing the converted synthesis gas stream into a sorption apparatus for removal of acidic gas constituents, especially carbon dioxide, by means of a physical or chemical sorption process, discharging a deacidified synthesis gas stream and an acid gas stream containing acidic gas constituents from the sorption apparatus, discharging the acid gas stream from the process,
(j) introducing at least a portion of the deacidified synthesis gas stream into a liquid nitrogen scrubbing stage, separating the deacidified synthesis gas stream in the liquid nitrogen scrubbing stage into the following substreams:
(j1) an ammonia synthesis feed stream containing hydrogen and nitrogen as main constituents and carbon monoxide and inert components as trace constituents,
(j2) a second residual gas stream containing hydrogen and carbon monoxide as main constituents and inert components as trace constituents,
(j3) an inert gas stream which contains inert components as the main constituent and is discharged from the process,
(k) introducing the ammonia synthesis feed stream into an ammonia synthesis reactor, at least partially converting the ammonia synthesis feed stream in the ammonia synthesis reactor under ammonia synthesis conditions, discharging an ammonia product stream from the ammonia synthesis reactor,
(l) introducing at least a portion of the methanol synthesis purge stream into the sorption apparatus,
(m) introducing into the methanol synthesis reactor one or more gas streams selected from the following group of:
(m1) a portion of the converted synthesis gas stream from process step (h)
(m2) a portion of the deacidified synthesis gas stream from process step (i)
(m3) at least a portion of the second residual gas stream from process step (j2).

2. Process according to Claim 1, **characterized in that** the synthesis gas production plant comprises
(b2) an autothermal reforming stage (ATR) or
(b3) a partial oxidation stage (POX) or
(b4) a combination of the stages (b2) to (b3) with one another or with a steam reforming stage heated using burners and/or hot gases
and **in that** the raw synthesis gas stream produced has a stoichiometry number of less than 2.

3. Process according to Claim 2, **characterized in that** the raw synthesis gas stream produced has a pressure of 40 bara or more, preferably 50 bara or more, most preferably 60 bara or more.

4. Process according to Claim 3, **characterized in that** the sorption apparatus operates by means of a physical absorption process and **in that** the sorption apparatus is at the same pressure level as the synthesis gas production plant.

5. Process according to Claim 4, **characterized in that** the sorption apparatus operates by means of gas scrubbing with cold methanol and **in that** a carbon dioxide-rich stream having a CO₂ content of at least 98% by volume, preferably at least 99% by volume, most preferably at least 99.5% by volume, is discharged from the sorption apparatus.

6. Process according to Claim 5, **characterized in that** the carbon dioxide-rich stream discharged from the sorption apparatus is sent to a CO₂ capture and storage process (CCS) and/or to a process for material utilization of carbon dioxide.

7. Process according to any of the preceding claims, **characterized in that** the one or more gas stream(s) introduced into the methanol synthesis reactor are adjusted, based on their molar flow, such that the stoichiometry number of the sum of the feed streams entering the methanol synthesis reactor is at least 2 or more, preferably at least 2.1 or more.

8. Process according to any of the preceding claims, **characterized in that** two or more gas streams are simultaneously introduced into the methanol synthesis reactor, wherein the two or more gas streams comprise: (m3) at least a portion of the second residual gas stream from process step (j2) and in addition one or more further gas streams selected from the following group of:
(m1) a portion of the converted synthesis gas stream from process step (h)
(m2) a portion of the deacidified synthesis gas stream from process step (i).

9. Plant for producing methanol and ammonia from an input stream containing hydrocarbons comprising the following components and constituents in fluid connection with one another:
(a) means for providing the input stream containing hydrocarbons,
(b) a synthesis gas production plant, means for supplying the input stream containing hydrocarbons to the synthesis gas production plant, wherein the synthesis gas production plant comprises:
(b1) a steam reforming stage heated using burners and/or hot gases or
(b2) an autothermal reforming stage (ATR) or
(b3) a partial oxidation stage (POX) or
(b4) a combination of at least two of the stages (b1) to (b3),
(c) means for discharging a raw synthesis gas stream containing hydrogen (H₂), carbon monoxide (CO) and inert components such as methane (CH₄) from the synthesis gas production plant and means for dividing the raw synthesis gas stream into a first raw synthesis gas substream and into a second raw synthesis gas substream,
(d) a methanol synthesis reactor, means for introducing at least a portion of the first raw synthesis gas substream into a methanol synthesis reactor,
(e) means for discharging a methanol-containing first reactor product stream from the methanol synthesis reactor, means for cooling the first reactor product stream to below its dew point, a phase separation apparatus, means for separating the cooled first reactor product stream in the phase separation apparatus into a first liquid product stream and a first residual gas stream containing unconverted synthesis gas constituents and inert components, means for discharging the first liquid product stream from the process as a raw methanol product stream,
(f) means for dividing the first residual gas stream into a methanol synthesis purge stream and into a recycle stream which is recycled to the methanol synthesis reactor,
(g) a CO conversion plant comprising at least one CO conversion stage, means for introducing at least a portion of the second raw synthesis gas substream into the CO conversion plant, means for discharging a converted synthesis gas stream from the CO conversion plant,
(h) a sorption apparatus for removal of acidic gas constituents, especially carbon dioxide, by means of a physical or chemical sorption process, means for introducing the converted synthesis gas stream into the sorption apparatus, means for discharging a deacidified synthesis gas stream and an acid gas stream containing acidic gas constituents from the sorption apparatus, means for discharging the acid gas stream from the process,
(i) a liquid nitrogen scrubbing stage, means for introducing at least a portion of the deacidified synthesis gas stream into the liquid nitrogen scrubbing stage, means for separating the deacidified synthesis gas stream in the liquid nitrogen scrubbing stage into the following substreams:
(i1) an ammonia synthesis feed stream containing hydrogen and nitrogen as main constituents and carbon monoxide and inert components as trace constituents,
(i2) a second residual gas stream containing hydrogen and carbon monoxide as main constituents and inert components as trace constituents,
(i3) an inert gas stream containing inert components as the main constituent,
(j) an ammonia synthesis reactor, means for introducing the ammonia synthesis feed stream into the ammonia synthesis reactor, means for discharging an ammonia product stream from the ammonia synthesis reactor,
(k) means for introducing into the methanol synthesis reactor one or more gas streams, wherein the gas streams are selected from the following group of:
(m1) a portion of the converted synthesis gas stream from process step (h)
(m2) a portion of the deacidified synthesis gas stream from process step (i)
(m3) at least a portion of the second residual gas stream from process step (j2).

10. Plant according to Claim 9, **characterized in that** the synthesis gas production plant comprises:
(b2) an autothermal reforming stage (ATR) or
(b3) a partial oxidation stage (POX) or
(b4) a combination of the stages (b2) to (b3) with one another or with a steam reforming stage heated using burners and/or hot gases.

11. Plant according to Claim 10, **characterized in that** it comprises at least one compression stage which allows the raw synthesis gas stream produced to have a pressure of 40 bara or more, preferably 50 bara or more, most preferably 60 bara or more.

12. Plant according to Claim 11, **characterized in that** the sorption apparatus operates by means of a physical absorption process and is configured such that the sorption apparatus is at the same pressure level as the synthesis gas production plant.

13. Plant according to Claim 12, **characterized in that** the sorption apparatus operates by means of gas scrubbing with cold methanol and is configured such that a carbon dioxide-rich stream having a CO₂ content of at least 98% by volume, preferably at least 99% by volume, most preferably at least 99.5% by volume, is discharged from the sorption apparatus.

14. Plant according to Claim 13, **characterized in that** it comprises means which allow the carbon dioxide-rich stream discharged from the sorption apparatus to be sent to a CO₂ capture and storage process (CCS) and/or to a process for material utilization of carbon dioxide.

15. Plant according to any of the preceding claims, **characterized in that** it comprises means which allow the one or more gas streams introduced into the methanol synthesis reactor to be adjusted, based on their molar flow, such that the stoichiometry number of the sum of the feed streams entering the methanol synthesis reactor is at least 2 or more, preferably at least 2.1 or more.

## Revendications

1. Procédé de production de méthanol et d'ammoniac à partir d'un courant de charge contenant des hydrocarbures, de préférence du méthane, tout spécialement du gaz naturel ayant une teneur en méthane d'au moins 80 % en volume, comprenant les étapes suivantes :
(a) fourniture du courant de charge contenant des hydrocarbures,
(b) amenée du courant de charge contenant des hydrocarbures à une installation de production de gaz de synthèse, comprenant
(b1) un étage de vaporeformage chauffé à l'aide de brûleurs et/ou de gaz chauds ou
(b2) un étage de reformage autotherme (ATR) ou
(b3) un étage d'oxydation partielle (POX) ou
(b4) une combinaison d'au moins deux des étages (b1) à (b3),
(c) réaction au moins partielle du courant de charge contenant des hydrocarbures dans l'installation de production de gaz de synthèse dans des conditions de production de gaz de synthèse, pour obtenir un courant de gaz de synthèse brut contenant de l'hydrogène (H₂), du monoxyde de carbone (CO) et des composants inertes tels que le méthane (CH₄),
(d) évacuation de l'installation de production de gaz de synthèse d'un courant de gaz de synthèse brut et division du courant de gaz de synthèse brut en un premier courant partiel de gaz de synthèse brut et un deuxième courant partiel de gaz de synthèse brut,
(e) introduction d'au moins une partie du premier courant partiel de gaz de synthèse brut dans un réacteur de synthèse du méthanol, réaction au moins partielle du premier courant partiel de gaz de synthèse brut dans le réacteur de synthèse du méthanol dans les conditions de synthèse du méthanol,
(f) évacuation du réacteur de synthèse du méthanol d'un premier courant de produits du réacteur contenant du méthanol, refroidissement du premier courant de produits du réacteur en dessous de son point de rosée et séparation du premier courant de produit du réacteur refroidi dans un dispositif de séparation de phases en un premier courant de produits liquides et un premier courant de gaz résiduel, qui contient des constituants du gaz de synthèse n'ayant pas réagi et des composants inertes, évacuation du premier courant de produits obtenu par le procédé, en tant que courant de produits de méthanol brut,
(g) division du premier courant de gaz résiduel en un courant de rinçage de synthèse du méthanol et en un courant de recyclage, qui est renvoyé au réacteur de synthèse du méthanol,
(h) introduction d'au moins une partie du deuxième courant partiel de gaz de synthèse brut dans une installation de conversion du CO, comprenant au moins un étage de conversion du CO, réaction de la partie du deuxième courant partiel de gaz de synthèse brut introduit dans l'installation de conversion du CO dans les conditions de conversion du CO pour obtenir un courant de gaz de synthèse converti, évacuation du courant de gaz de synthèse converti,
(i) introduction du courant de gaz de synthèse converti dans un dispositif de sorption pour éliminer les constituants gazeux acides, en particulier le dioxyde de carbone, à l'aide d'un procédé de sorption physique ou chimique, évacuation du dispositif de sorption d'un courant de gaz de synthèse désacidifié et d'un courant de gaz acide contenant des constituants gazeux acides, évacuation d procédé du courant de gaz acide,
(j) introduction d'au moins une partie du courant de gaz de synthèse désacidifié dans un étage de lavage à l'azote liquide, séparation du courant de gaz de synthèse désacidifié dans l'étage de lavage à l'azote liquide en les courants partiels suivants :
(j1) un courant de charge de synthèse d'ammoniac contenant de l'hydrogène et de l'azote en tant que constituants principaux et du monoxyde de carbone et des composants inertes en tant que constituants en traces,
(j2) un deuxième courant de gaz résiduel contenant de l'hydrogène et du monoxyde de carbone en tant que constituants principaux et des composants inertes en tant que constituants en traces,
(j3) un courant de gaz inerte contenant des composants inertes en tant que constituant principal, qui est évacué du procédé,
(k) introduction du courant de charge de synthèse d'ammoniac dans un réacteur de synthèse d'ammoniac, réaction au moins partielle du courant de charge de synthèse d'ammoniac dans le réacteur de synthèse d'ammoniac dans les conditions de synthèse d'ammoniac, évacuation du réacteur de synthèse d'ammoniac d'un courant d'ammoniac produit,
(l) introduction d'au moins une partie du courant de rinçage de synthèse du méthanol dans le dispositif de sorption,
(m) introduction d'un ou plusieurs courants gazeux dans le réacteur de synthèse du méthanol, qui sont choisis dans le groupe suivant :
(m1) une partie du courant de gaz de synthèse converti de l'étape (h)
(m2) une partie du courant de gaz de synthèse désacidifié de l'étape (i)
(m3) au moins une partie du deuxième courant de gaz résiduel de l'étape (j2).

2. Procédé selon la revendication 1, **caractérisé en ce que** l'installation de production de gaz de synthèse comprend
(b2) un étage de reformage autotherme (ATR) ou
(b3) un étage d'oxydation partielle (POX) ou
(b4) une combinaison des étages (b2) à (b3) l'un à l'autre ou à un étage de vaporeformage chauffé à l'aide de brûleurs et/ou de gaz chauds,
et **en ce que** le courant de gaz de synthèse brut produit présente un indice stœchiométrique inférieur à 2.

3. Procédé selon la revendication 2, **caractérisé en ce que** le courant de gaz de synthèse brut produit présente une pression de 40 bara ou plus, de préférence de 50 bara ou plus, tout spécialement de 60 bara ou plus.

4. Procédé selon la revendication 3, **caractérisé en ce que** le dispositif de sorption travaille par un procédé d'absorption physique, et **en ce que** le dispositif de sorption se trouve au même niveau de pression que l'installation de production de gaz de synthèse.

5. Procédé selon la revendication 4, **caractérisé en ce que** le dispositif de sorption travaille par lavage du gaz avec du méthanol froid, et **en ce qu'**un courant riche en dioxyde de carbone est évacué du dispositif de sorption, courant dont la teneur en CO₂ est d'au moins 98 % en volume, de préférence d'au moins 99 % en volume, tout spécialement d'au moins 99,5 % en volume.

6. Procédé selon la revendication 5, **caractérisé en ce que** le courant riche en dioxyde de carbone, évacué du dispositif de sorption, est envoyé à un procédé de captage et de stockage du CO₂ (CCS) et/ou à un procédé d'utilisation matérielle de dioxyde de carbone.

7. Procédé selon l'une des revendications précédentes, **caractérisé en ce que** le ou les courants gazeux qui sont introduits dans le réacteur de synthèse du méthanol sont, pour ce qui est de leur débit massique, ajustés de telle sorte que l'indice stœchiométrique de la somme des courants de charge pénétrant dans le réacteur de synthèse du méthanol soit d'au moins 2 ou plus, de préférence d'au moins 2,1 ou plus.

8. Procédé selon l'une des revendications précédentes, **caractérisé en ce que** plusieurs courants gazeux sont simultanément introduits dans le réacteur de synthèse du méthanol, les plusieurs courants gazeux comprenant : (m3) au moins une partie du deuxième courant de gaz résiduel de l'étape (j2) et en outre un ou plusieurs autres courants gazeux, qui sont choisis dans le groupe suivant :
(m1) une partie du courant de gaz de synthèse converti de l'étape (h)
(m2) une partie du courant de gaz de synthèse désacidifié de l'étape (i).

9. Installation de production de méthanol et d'ammoniac à partir d'un courant de charge contenant des hydrocarbures, comprenant les composants et constituants suivants, en liaison fluidique les uns avec les autres :
(a) des moyens pour fournir le courant de charge contenant des hydrocarbures,
(b) une installation de production de gaz de synthèse, des moyens pour amener le courant de gaz de synthèse contenant des hydrocarbures à l'installation de production de gaz de synthèse, l'installation de production de gaz de synthèse comprenant :
(b1) un étage de vaporeformage chauffé à l'aide de brûleurs et/ou de gaz chauds ou
(b2) un étage de reformage autotherme (ATR) ou
(b3) un étage d'oxydation partielle (POX) ou
(b4) une combinaison d'au moins deux des étages (b1) à (b3),
(c) des moyens pour évacuer de l'installation de production de gaz de synthèse un courant de gaz de synthèse brut contenant de l'hydrogène (H₂), du monoxyde de carbone (CO) et des composants inertes tels que le méthane (CH₄), et des moyens pour diviser le courant de gaz de synthèse brut en un premier courant partiel de gaz de synthèse brut et un deuxième courant partiel de gaz de synthèse brut,
(d) un réacteur de synthèse du méthanol, des moyens pour introduire au moins une partie du premier courant partiel de gaz de synthèse dans un réacteur de synthèse du méthanol,
(e) des moyens pour évacuer du réacteur de synthèse du méthanol un premier courant de produit de réacteur contenant du méthanol, des moyens pour refroidir le premier courant de produit de réacteur en dessous de son point de rosée, un dispositif de séparation des phases, des moyens pour séparer le premier courant de produit de réacteur refroidi se trouvant dans le dispositif de séparation des phases dans un premier courant de produit liquide et un premier courant de gaz résiduel, qui contient des constituants du gaz de synthèse n'ayant pas réagi et des composants inertes, des moyens pour évacuer le premier courant de produit liquide du procédé en tant que courant de produit de méthanol brut,
(f) des moyens pour diviser le premier courant de gaz partiel en un courant de rinçage de synthèse du méthanol et un courant de recyclage, qui est renvoyé au réacteur de synthèse du méthanol,
(g) une installation de conversion de CO, comprenant au moins un étage de conversion de CO, des moyens pour introduire au moins une partie du deuxième courant partiel de gaz de synthèse brut dans l'installation de conversion de CO, des moyens pour évacuer de l'installation de conversion de CO un courant de gaz de synthèse converti,
(h) une installation de sorption, pour éliminer les constituants gazeux acides, en particulier le dioxyde de carbone, à l'aide d'un procédé de sorption physique ou chimique, des moyens pour introduire le courant de gaz de synthèse converti dans le dispositif de sorption, des moyens pour évacuer du dispositif de sorption un courant de gaz de synthèse désacidifié et un courant de gaz acide contenant des constituants gazeux acides, des moyens pour évacuer du procédé le courant de gaz acide,
(i) une installation de lavage à l'azote liquide, des moyens pour introduire au moins une partie du courant de gaz de synthèse désacidifié dans l'étage de lavage à l'azote liquide, des moyens pour séparer le courant de gaz de synthèse désacidifié se trouvant dans l'étage de lavage à l'azote liquide en les courants partiels suivants :
(i1) un courant de charge de synthèse d'ammoniac contenant de l'hydrogène et de l'azote en tant que constituants principaux et du monoxyde de carbone et
des composants inertes en tant que constituants en traces,
(i2) un deuxième courant de gaz résiduel contenant de l'hydrogène et du monoxyde de carbone en tant que constituants principaux et des composants inertes en tant que constituants en traces,
(i3) un courant de gaz inerte contenant des composants inertes en tant que constituant principal,
(j) un réacteur de synthèse d'ammoniac, des moyens pour introduire dans le réacteur de synthèse d'ammoniac le courant de charge de synthèse d'ammoniac, des moyens pour évacuer du réacteur de synthèse d'ammoniac un courant de produit d'ammoniac,
(k) des moyens pour introduire dans le réacteur de synthèse du méthanol un ou plusieurs courants gazeux, les courants gazeux étant choisis dans le groupe suivant :
(m1) une partie du courant de gaz de synthèse converti de l'étape (h)
(m2) une partie du courant de gaz de synthèse désacidifié de l'étape (i)
(m3) au moins une partie du deuxième courant de gaz résiduel de l'étape (j2).

10. Installation selon la revendication 9, **caractérisée en ce que** l'installation de production de gaz de synthèse comprend :
(b2) un étage de reformage autotherme (ATR) ou
(b3) un étage d'oxydation partielle (POX) ou
(b4) une combinaison des étages (b2) à (b3) l'un à l'autre ou à un étage de vaporeformage chauffé à l'aide de brûleurs et/ou de gaz chauds.

11. Installation selon la revendication 10, **caractérisée en ce qu'**elle comprend au moins un étage de compression, qui permet que le courant de gaz de synthèse brut produit présente une pression de 40 bara ou plus, de préférence de 50 bara ou plus, tout spécialement de 60 bara ou plus.

12. Installation selon la revendication 11, **caractérisée en ce que** le dispositif de sorption travaille à l'aide d'un procédé d'absorption physique et est conçu de façon que le dispositif de sorption se trouve au même niveau de pression que l'installation de production de gaz de synthèse.

13. Installation selon la revendication 12, **caractérisée en ce que** le dispositif de sorption travaille à l'aide d'un lavage du gaz au méthanol froid et est conçu de façon qu'un courant riche en dioxyde de carbone soit évacué du dispositif de sorption, courant dont la teneur en CO₂ est d'au moins 98 % en volume, de préférence d'au moins 99 % en volume, tout spécialement d'au moins 99,5 % en volume.

14. Installation selon la revendication 13, **caractérisée en ce qu'**elle comprend des moyens qui permettent que le courant riche en dioxyde de carbone évacué du dispositif de sorption soit envoyé à un procédé de captage et de stockage du CO₂ (CCS) et/ou à un procédé d'utilisation matérielle du dioxyde de carbone.

15. Installation selon l'une des revendications précédentes, **caractérisée en ce qu'**elle comprend des moyens qui permettent que le ou les courants gazeux qui sont introduits dans le réacteur de synthèse soient, pour ce qui est de leur débit massique, ajustés de telle sorte que l'indice stœchiométrique de la somme des courants de charge pénétrant dans le réacteur de synthèse du méthanol soit d'au moins 2 ou plus, de préférence d'au moins 2,1 ou plus.
